# EUROPEAN PATENT SPECIFICATION

(11) **EP 2 566 396 B1**
(45) Date of publication and mention of the grant of the patent: **30.04.2014**
(21) Application number: 10726228.9
(22) Date of filing: 05.05.2010
(51) Int. Cl.: A61B 17/00, A61M 25/04, A61M 1/00

(54) **DEVICE FOR SEALING PERFORATIONS AND SUSTAINING FLOW**
VORRICHTUNG ZUR VERSIEGELUNG VON PERFORATIONEN UND ZUR AUFRECHTERHALTUNG EINES FLUSSES
DISPOSITIF DESTINÉ À ASSURER L'ÉTANCHÉITÉ DE PERFORATIONS ET MAINTENIR UN ÉCOULEMENT

(43) Date of publication of application: 13.03.2013
(73) Proprietor: Deliverance Ltd., 12900 Kazerin (IL)
(72) Inventor: ARAVOT, Dan, 12900 Katzerin (IL); ALMON, Ehud, 36089 Kiryat Tivon (IL); HIRSZOWICZ, Eran, 52236 Ramat Gan (IL); YARON-BRAUMAN, Hila, 69101 Tel-Aviv (IL)
(74) Representative: Messulam, Alec Moses
(86) International application number: PCT/IB2010/051984
(87) International publication number: WO 2010/128469

(56) References cited:
- FR-A1- 2 607 706
- US-A1- 2006 161 110
- US-A1- 2006 190 036
- US-B1- 6 296 658

## Description

### FIELD OF THE INVENTION

The present invention relates to medical devices in general. More particularly, the present invention relates to devices capable of sealing perforations, inter alia within the cardiovascular system, and sustaining a flow thereto/therefrom.

### BACKGROUND ART

One of the main causes of death in trauma injuries is haemorrhage from the major blood vessels of the torso or the heart. If not efficiently treated without delay, the haemorrhage causes exsanguination and may lead to death within minutes. Exsanguination is the estimated main cause in 5-10% of the death toll of urban trauma injuries and 10% or more in military trauma injuries.

Contradistinctively to the limbs, whereon a tourniquet or direct pressure can be employed in order to efficiently halt a haemorrhage, perforations or ruptures in the major blood vessels of the torso or the heart itself cannot be treated in either of these manners. Furthermore, if the rupture occurs near the bifurcation of a limb from the corpus, such as for instance rupture in the femoral artery at the groin region, a tourniquet cannot be used and a direct pressure may fail to achieve the desired result. Moreover, whenever a tourniquet is applicable, if employed for a prolonged time period, the accompanying interruption of blood circulation in the limb may occasionally lead to necrosis of the limb tissues and necessitate amputation.

A device for sealing perforations by discretely blocking the perforation itself, from inside and outside of the vessel's wall around the perforation, rather than by means of a direct or radial pressure and complete cave-in of the blood vessel would be applicable in treating perforations or ruptures of the major blood vessels of the corpus and of the heart. Such a device would be specifically beneficial in treating haemorrhages associated with the blood vessels of the thoracic, abdominal and pelvic sections of the corpus such as the aorta, abdominal aorta, vena cava, pulmonary vein, thoracic aorta, etc. Moreover, such a device would also constitute a preferred alternative for treating haemorrhages of blood vessels of the limbs, allowing halting a haemorrhage and maintaining blood circulation in the limb.

A specific class of trauma is the substantial haemorrhage from injured major blood vessels of the corpus or the heart. This is typically accompanied by hypovolemia which requires a rapid restitution of the volume of circulating blood in the body. Pressure infusion devices (PIDs) such as various sleeves, jackets, pumps and the device commercialized under the tradename of Level 1 (Level 1 Technologies, Inc., Rockland, MA) are widely used to maintain normovolemia in patients experiencing large-volume blood loss. Whereas the prevailing, routinely used PIDs can infuse liquids with flow rates of up-to ~200 mL/min, the Level 1 devices can uphold flow rates of up to 500 mL/min. Such flow rates may nevertheless be insufficient for effective treatment of severe trauma injuries. High-Flow systems, employing canulas with diameter of up to 10 mm, such as the High-Flow Blood Filter (Saftifilter Blood Administration Sets; Cutter Biological, Berkeley, CA) can sustain flow rates of up to ~600-1500 mL/min; however, these systems are not portable and thus typically cannot be used for treatment of trauma conditions. A portable, handy device applicable in emergency field-care of injury incidents and capable of infusing liquids with flow rates of more than 500 mL/min, would thus have a specific benefit in emergency treatment of trauma conditions.

US patents Ser. Nos. 7169176, 7011679, 6976952, 6740111, 6635080, 4747848, 4545082, 6368347, 6352554 and 6264662 are believed to represent the current state of the art.

Document FR 2 607 706 discloses a medical device for sealing perforations comprising a catheter member comprising at least an expandable flange at the distal end thereof, wherein said flange is capable of assuming an unfolded conformation, a cannula member, wherein said cannula member is disposable over said catheter member; and a sealing element.

### SUMMARY OF THE INVENTION

In accordance with the present invention a device capable of sealing perforation/s within and sustaining a flow into or out of an organ according to claim 1 is provided. The device is specifically beneficial in emergency trauma care of perforations or raptures in the cardiovascular system. The device can be beneficially employed for performing a quick aortic cannulation procedure or other catheterizations.

The device of the present invention is a dual-purpose tool. Each one of its functions is associated with specific structural features, as will be described below in some detail. The object of the first function is to seal perforations, e.g. in the cardiovascular system caused by trauma injuries and characterized by a substantial haemorrhage. The object of the second function is to sustain effective amounts of liquid flow into the sealed organ or the drainage of fluids therefrom; in the case of cardiovascular system, in order to avoid possible hypovolemic shock caused by the haemorrhage, thereby stabilizing the condition of the patient.

### BRIEF DESCRIPTION OF THE DRAWINGS

The present invention will be understood and appreciated more fully from the following detailed description taken in conjunction with the appended drawings in which:
- **Fig. 1A**: is an isometric view of an embodiment of the device for sealing perforations and sustaining a flow of liquids;
- **Fig. 1B**: is an exploded isometric view of a device for sealing perforations and sustaining a flow of liquids;
- **Fig. 2A**: is an enlarged isometric view of the anterior portion the catheter member and the expandable flange, in sprawled or unfolded conformation, of the device shown in **Figs 1A** and **1B**;
- **Fig. 2B**: is an enlarged cross-sectional view of the anterior portion the catheter member and the expandable flange, in sprawled or unfolded conformation, of the device shown in **Figs** 1**A** and **1B**;
- **Fig. 2C**: is an enlarged cross-sectional view of the expandable flange, in contacted or folded conformation, disposed within the confining sleeve;
- **Fig. 3A**: is an isometric view of the cannula member, of the device shown in **Figs 1A** and **1B**;
- **Fig. 3B**: is an isometric view of the pulling tab of the cannula member shown in **Fig. 3A**;
- **Fig. 3C**: is an isometric view of the confining sleeve of the cannula member shown in **Fig. 3A**;
- **Fig. 3D**: **is** a side view of the confining sleeve of the cannula member shown in **Fig. 3A**;
- **Fig. 4A**: **is** an isometric view of the sealing element, of the device shown in **Figs 1A** and **1B**;
- **Fig. 4B**: is an exploded isometric view of the sealing element, of the device shown in **Figs 1A** **and** **1B**;
- **Fig. 4C**: is a cross-sectional view of the toroidal cushion of the sealing element shown in **Figs 4A** and **4B**;
- **Fig. 5**: is an isometric view of a preferred embodiment device for sealing perforations and sustaining a flow of liquids;
- **Fig. 6A**: is a cross-sectional view of the posterior portion of the device for sealing perforations and sustaining a flow of liquids shown in **Fig. 5**;
- **Fig. 6B**: is an enlarged cross-sectional view of the locking mechanism of the device shown in **Fig. 6A**, in inactive configuration;
- **Fig. 6C**: is a cross-sectional view of the posterior portion of the device for sealing perforations and sustaining a flow of liquids shown in **Fig. 5**;
- **Fig. 6D**: is an enlarged cross-sectional view of the locking mechanism of the device shown in **Fig. 6C**, in active configuration;
- **Fig 7A**: is an isometric view of configuration **300A** of an embodiment of the device of the present invention adapted for sealing perforations in globular or apical organs;
- **Fig 7B**: is an isometric view of configuration **300B** of an embodiment of the device of the present invention adapted for sealing perforations in globular or apical organs;
- **Fig 7C**: is an isometric view of configuration **300C** of an embodiment of the device of the present invention adapted for sealing perforation in globular or apical organs;
- **Fig 8**: is an isometric view of yet another embodiment **400** of the device of the present invention adapted for sealing perforation in globular or apical organs.

### DISCLOSURE OF THE INVENTION

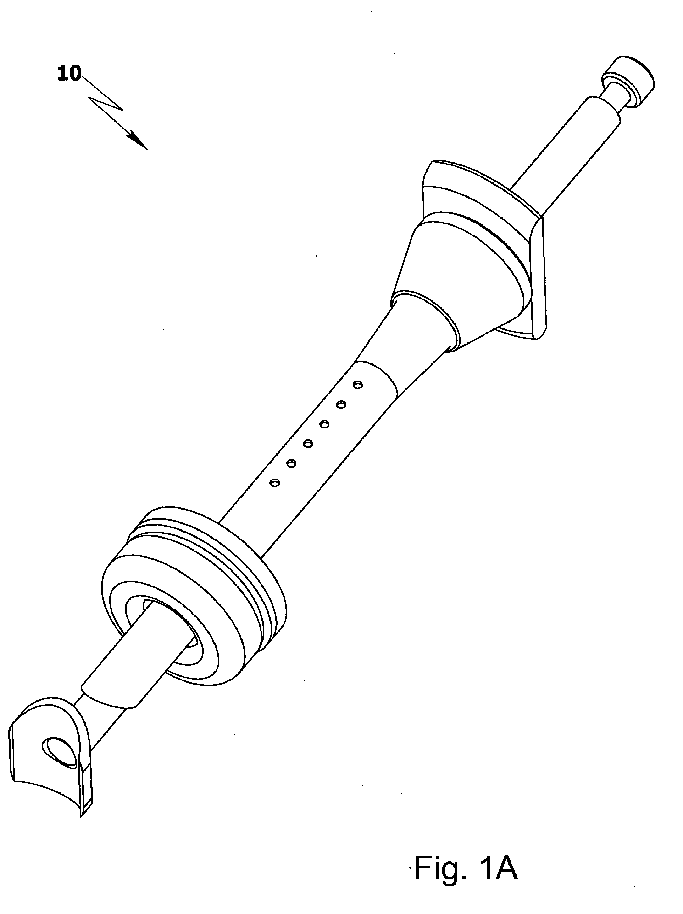

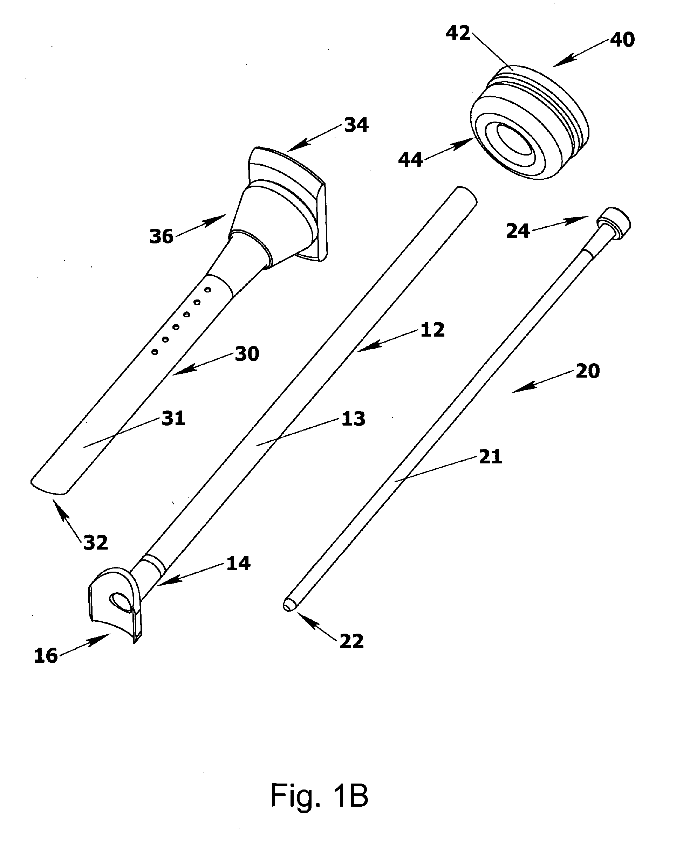

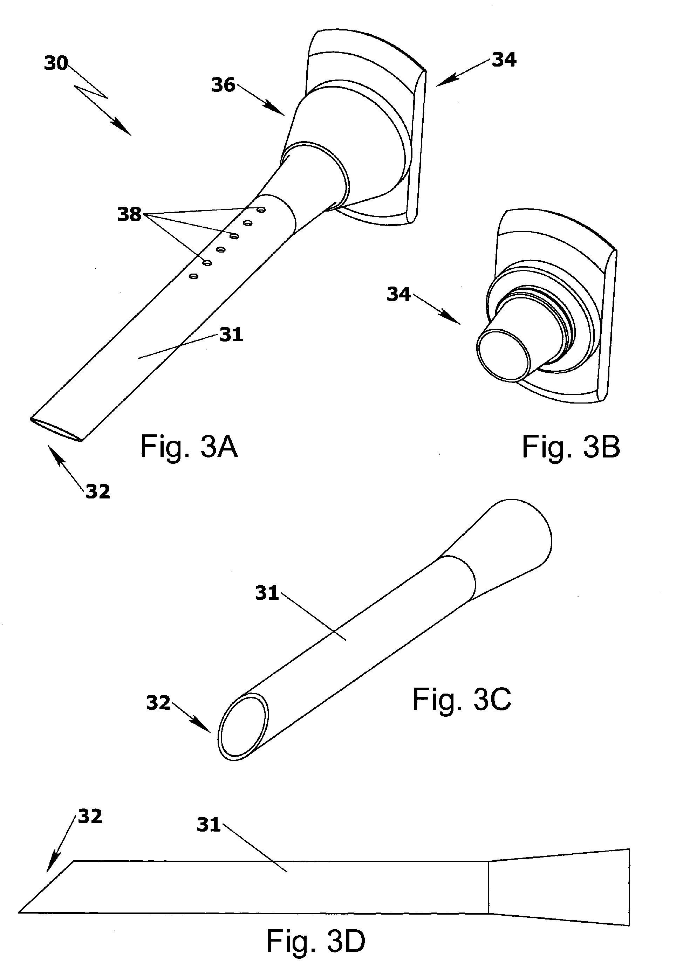

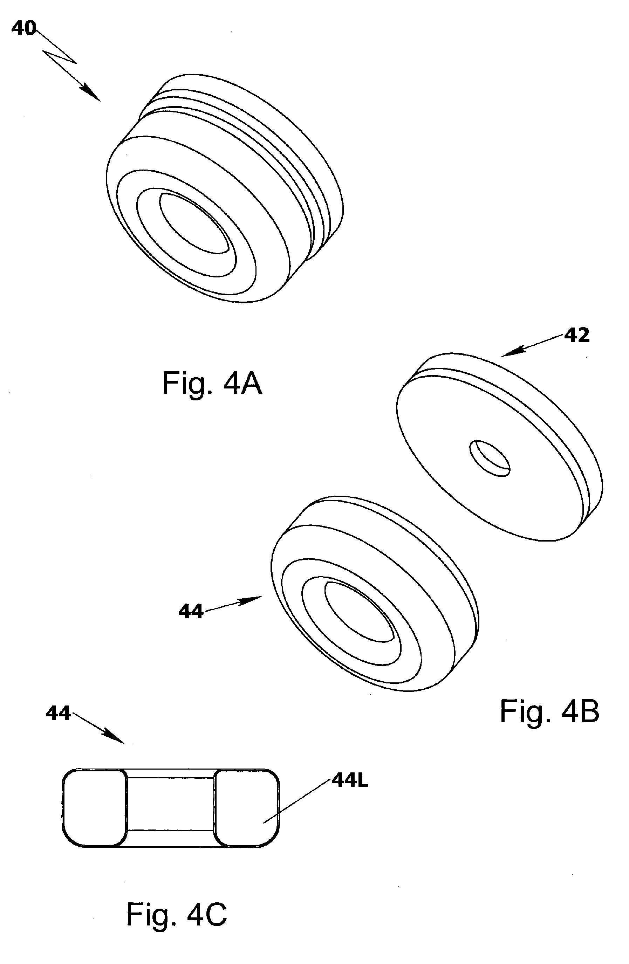

Reference is now made to **Figs 1** to **4****,** showing device **10** for sealing perforations and sustaining a flow of liquids (hereinafter DSPSFL). DSPSFL **10** is adapted for sealing perforations in essentially tubular organs or tracts, e.g. various organs of the cardiovascular system and particularly blood vessels. DSPSFL **10** comprises catheter member **12,** mandrel member **20,** cannula member **30** and sealing element **40.**

Catheter member **12** comprises sheath element **13,** furnished with thin-walled neck portion **14** towards distal end, and terminates with expandable flange **16** at the distal or otherwise anterior end; the end of member **12** opposite flange **16** is referred to hereinafter as proximal or posterior. Mandrel member **20** comprises core shaft **21,** distal tip **22** and proximal cap **24.** Cannula member **30** comprises confining sleeve **31,** terminated by tip **32,** pulling tab **34** and interconnecting element **36.** Sealing element **40** comprises bushing **42** and toroidal cushion **44.**

Sheath element **13** comprises interior lumen **13L** extending therethrough. The outer diameter of sheath element **13** typically ranges from about 3mm to about 8mm but the range of about 6 to 7mm is preferable. The inner diameter of sheath element **13** typically ranges from about 1.5mm to about 7mm but the preferable range is about 4.5 to 6 mm. Sheath element **13** is preferably made of biocompatible elastomeric material, such as silicone rubber or soft PeBax^{®}. The material that sheath element **13** made of is preferably characterized by Shore hardness of about 60 units, on the A-type D2240 ASTM durometric scale, and elongation ability of about 350%, before reaching the yield point.

The outer diameter of neck portion **14** typically equals the outer diameter of sheath element **13.** The thickness of the wall of neck portion **14** is preferably less than 1 mm. Neck portion **14** is preferably characterized by Shore hardness of about 40 units, on the A-type D2240 ASTM scale, and elongation ability of about 750%, before reaching the yield point. Provided that sheath element **13** and neck portion **14** thereof are made of different materials various methods known in the art may be employed to form the integral structure of catheter-like member **12,** inter alia including co-molding and conjugation. The distal portion of sheath element **13** or neck portion **14** of member **12** may be furnished with notches, recesses or grooves to facilitate affixing of sealing element **40** at the anterior of sealing element **40,** as will be elaborated infra.

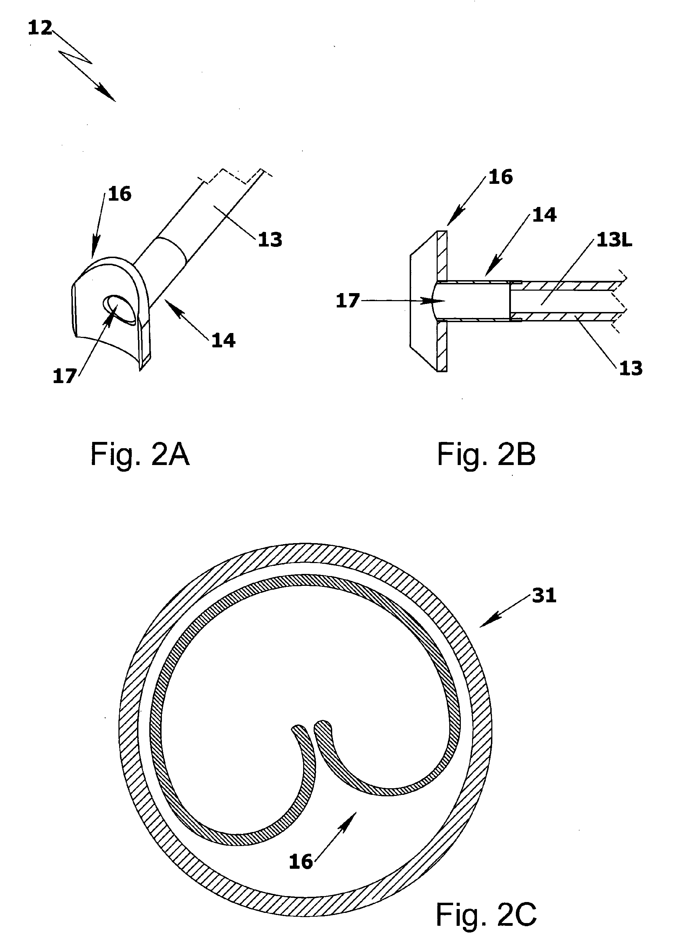

In some examples expandable flange **16** is characterized by a trough-shaped configuration, with chamfered or rounded edges and/or corners. The convex surface of expandable flange **16** is adapted to adjoin the interior surface of a blood vessel having an essentially tubular shape. The width of expandable flange **16** typically ranges from 10mm to 15mm and lengths the form 15mm to 30mm. Referring now to **Fig. 2A****,** expandable flange **16** comprises aperture **17,** at the centre thereof, forming a continuum with interior lumen **13L** of sheath element **13.** Aperture **17** is also referred to hereinafter as the outlet of DSPSFL **10;** whereas the aperture at the proximal end of sheath element **13** is referred to as the inlet thereof.

It should be acknowledged that expandable flange **16** of a trough-shaped configuration and the sizes set forth supra is merely an exemplary element of an embodiment of the invention; whereas the expandable elements to be used with catheter-like member of the present invention may assume different shapes and a variety of sizes, mainly dependent upon the medical application in which the DSPSFL is employed and the organ on which the DSPSFL is implemented, as will be described below in some detail.

Expandable flange **16** is capable of assuming, inter alia, a contracted (folded) or an expanded (sprawled or unfolded) conformations. In some examples, expandable flange **16** is made entirely of a resilient material that tends to inherently expand when contracted, due to the intrinsic bias thereof. In other examples, expandable flange **16** is made of a less biasing material typically furnished with structural framework or scaffolding (not shown), for instance embedded at the rims thereof, which confer the desired intrinsic bias and the expandability to the expandable flange.

The conformation of expandable flange **16** shown in **Figs. 2A** and **2B** is the expanded or sprawled one; whereas in the contracted or folded conformation flange **16** is accommodated within confining sleeve **31.** Expandable flange **16** may be folded up, for instance so that the flanking portions thereof are folded vis-à-vis each other, as schematically shown in **Fig. 2C****.** Any other contracted or folded conformation of expandable flange **16** may be employed to facilitate the accommodation thereof within confining sleeve **31.** In contracted conformation flange **16** confined by sleeve **31;** thereby the expansion of the former is precluded by the latter. In contracted conformation the effective exterior diameter of DSPSFL **10** is the outer diameter of sleeve **31.**

Expandable flange **16** may further include at least one structural element (not shown), in a non-limiting manner including slot, aperture, tab or pocket, dedicated for engagement with tip **22** of mandrel **20;** whereby upon manipulating/advancing mandrel **20,** expandable flange **16** is respectively manipulated/advanced. The aforementioned structural element is typically disposed on the concave surface of expandable flange **16.**

Mandrel member **20** comprises core shaft **21.** Core shaft **21** is an essentially elongated cylindrical body, made of a rigid material, e.g. stainless steel and/or polymeric material. Core shaft **21** is insertable into lumen **13L** and used to confer structural firmness to sheath element **13;** thereby allowing manipulating/advancing of DSPSFL **10.**

Mandrel member **20** comprises tip **22** at the distal end thereof. Tip **22** may include at least one structural element (not shown), in a non-limiting manner including slot, aperture, tab or pocket, dedicated for engagement with a respective structural element (not shown) of expandable flange **16.**

Mandrel member **20** further comprises cap **24** at the proximal end thereof. Cap **24** can be shaped like a knob and/or furnished with texture to facilitate a more ergonomic and convenient grip thereof; thereby facilitating manipulation/advancement of DSPSFL **10** by the operator.

In some examples shaft **21** comprises a lumen (not shown) extending therethrough and tip **22** includes an aperture at the anterior end allowing the access of blood thereto. Cap **24** is transparent or at least translucent and comprises an interior lumen (not shown) forming a continuum with the lumen of shaft **21** (not shown). By this means, upon placement of DSPSFL **10** inside a blood vessel, tip **22** is exposed to the arterial blood pressure and the blood fills the lumen of shaft **21** (not shown) eventually partially occupying the interior of cap **24;** whereby the presence of the tip of DSPSFL **10** in a blood vessel can be visually detected (serving as a blood indicator), much in the same way as caps of the needles of venous cannulae commercialized under the tradename Venflon^{®}.

Cannula member **30** comprises confining sleeve **31.** Sleeve **31** terminates with slanted distal tip **32.** The outer diameter of sleeve **31** typically ranges from about 4mm to about 10mm; preferably from about 7mm to 8mm. The inner diameter of sleeve **31** typically equals the outer diameter of sheath element **13** or slightly larger so as to establish a contiguous sliding of the former along the latter. Sleeve **31** is preferably made of a stiff biocompatible polymeric material, such as PTFE (Teflon^{®}), PeBax , Nylon or a combination of several materials. Distal tip **32** is preferably shaped and sized as the tips of aortic cannulae known in the art. Distal tip **32** may be made of an elastomeric material softer than sleeve **31.** Pulling tab **34** is secured by interconnecting element **36** to sleeve **31;** thereby upon pulling tab **34,** sleeve **31** is retracted therewith.

Sleeve **31** of cannula member **30** confines expandable flange **16,** thereby precluding the unfolding, sprawling or expansion of the latter, thus retaining it in the contracted or folded conformation. Cannula member **30** is preferably designed essentially as aortic cannulae and adapted to facilitate the ease of manipulation thereof by the operator, so as to be conveniently placed within an organ, such as blood vessel or the heart, through a perforation or incision therein. Cannula member **30** may further include graduations or indicia **38,** to indicate the depth of the penetration into a perforation, incision or wound and the orientation of inclination of slanted tip **32** at the distal end of confining sleeve **31** while therein.

Referring to **Fig**s **4A-C,** sealing element **40** comprises bushing **42** and anteriorly disposed toroidal cushion **44.** Bushing **42** is contiguously slidable along catheter member **12.** The circumference of bushing **42** can be ergonomically shaped and/or furnished with texture to facilitate en enhanced grip thereof. Bushing **42** may further include a catches or detents (not shown), to be affixed thereby to the proximal end of catheter member **12,** upon interaction with the respective notches, recesses or grooves at the distal portion of sheath element **13** or neck portion **14** of member **12,** as were mentioned supra. The outer diameter of toroidal cushion **44** typically ranges from about 15mm to about 35mm and the thickness ranges from about 10mm to about 20mm. Toroidal cushion **44** comprises balloon-like lumen **44L.** Toroidal cushion **44** is preferably made of an elastomeric biocompatible material, such as silicone rubber. Lumen **44L** is filled with biocompatible gel of about 3 to 10 Shore hardness units, on the A-type D2240 ASTM durometric scale, such as silicone gel MED2-6300 available from NuSil, at 1105 Arlington Drive Toms River, NJ 08755 USA. In some examples lumen **44L** is filled with gaseous substance, e.g. air.

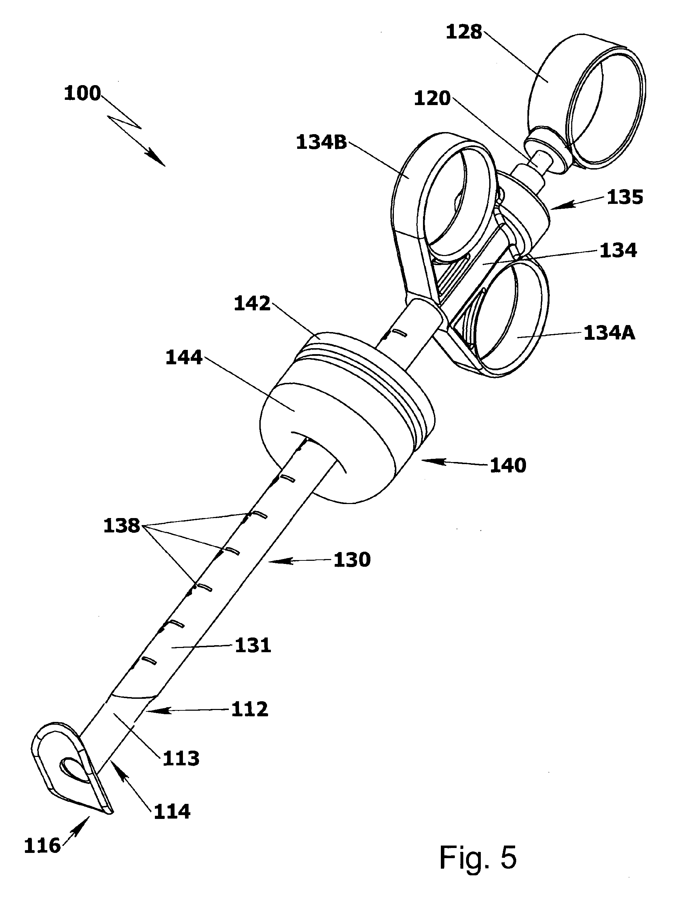

Reference is now made to **Figs 5** to **6****,** showing DSPSFL **100.** DSPSFL **100** is adapted for sealing perforations in essentially tubular organs, e.g. blood vessels. DSPSFL **10** comprises catheter member **12,** mandrel member **20,** cannula member **30** and sealing element **40.**

Catheter member **112** comprises sheath element **113,** furnished with thin-walled neck portion **114** towards distal end, and terminates with expandable flange **116.** Mandrel member **120** comprises proximal thumb-hold tab **128.** Cannula member **130** comprises confining sleeve **131** including indicia or graduations **138** and terminating with a slanted tip, pulling element **134** that includes finger-hold tabs **134A** and **134B** and mandrel interlocking mechanism **135.** Sealing element **140** comprises bushing **142** and toroidal cushion **144.** The aforementioned parts of DSPSFL **100** are characterized by reference to their respective equivalents and counterparts of DSPSFL **10,** unless otherwise indicated.

The proximal thumb-hold tab **128** of mandrel member **120** and finger-hold tabs **134A** and **134B** of pulling element **134** facilitate an enhanced ease of operation of DSPSFL **100.** Finger-hold tabs **134A** and **134B** particularly provide for posteriorly retracting pulling element **134** and confining sleeve **131** of the cannula member **130** over catheter member **112,** while concomitantly stabilizing mandrel **120** by grasping thumb-hold tab **128,** which is convenient for operating DSPSFL **100,** as elaborated below.

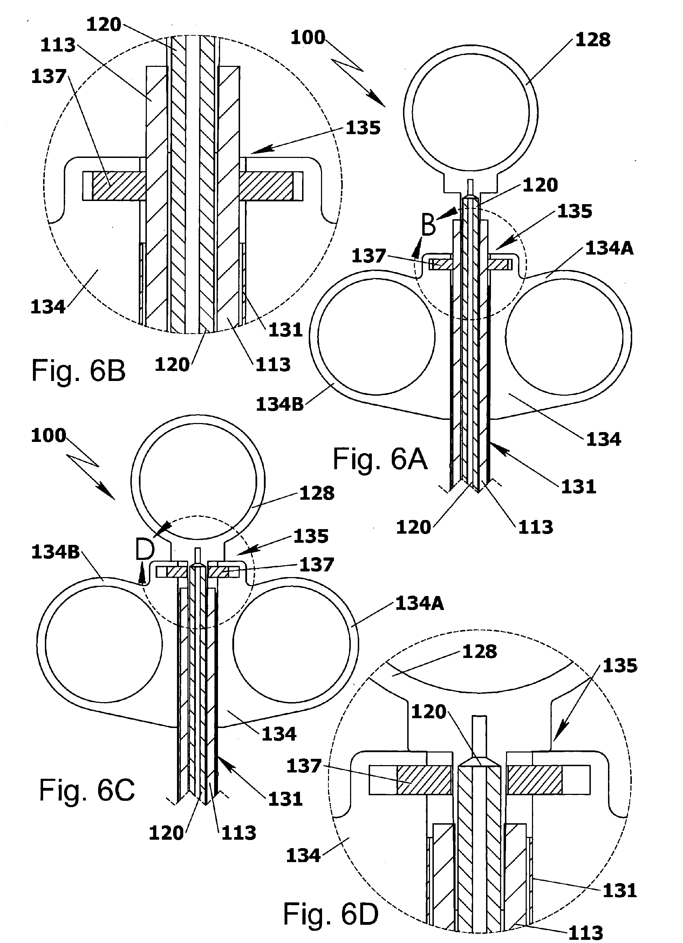

Mandrel interlocking mechanism **135** of cannula member **130** comprises annular locking element **137.** Locking element **137** is a split-ring or -washer or spring-washer or any other element capable of being biased towards small internal diameter. Interlocking mechanism **135** is employed to facilitate the removal of mandrel **120** from the interior lumen of sheath element **113** while concomitantly stabilizing catheter member **112** thus preventing any forceful pulling of sheath element **113** and particularly of expandable flange **116.** In inactive configuration annular locking element **137** is disposed around sheath element **113** contiguously slidable along the exterior surface thereof, as shown in **Fig. 6****B.** Upon translation of pulling element **134** in the posterior direction relatively to sheath element **113,** annular locking element **137** is slid over the exterior surface of the latter, until reaching the proximal end thereof. Thereupon annular locking element **137** spontaneously contracts to assume a diminished internal diameter, i.e. active configuration, shown in **Fig. 6D****.** In active the configuration, annular locking element **137** is disposed around mandrel **120** thereby effectively preventing posterior translation of sheath element **113** beyond annular locking element **137** and away from pulling element **134.** Accordingly, the operator can pull mandrel **120** from the interior lumen of sheath element **113,** for instance by applying a manual force to thumb-hold tab **128** in the posterior direction, while preventing translation of sheath element **113** in the posterior direction, by stabilizing pulling element **134,** for instance by grasping finger-hold tabs **134A** and **134B.**

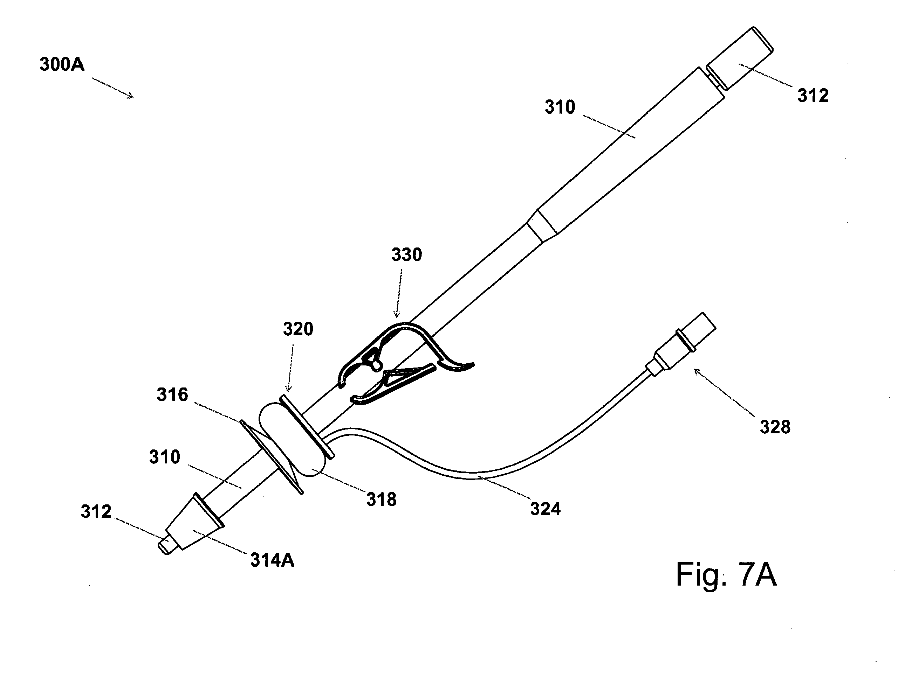

The examples of the DSPSFL shown throughout **Figs 1** to **6** are particularly adapted for sealing perforations in an essentially tubular organ, such as blood vessel. The method of operating the DSPSFL will be explained in more details below, by explaining in same detail the operation of DSPSFL implemented on globular or apical organs. To elaborate structural and functional feature of DSPSFL embodiments adapted for sealing perforations in an essentially globular or apical organ, e.g. the heart, reference is now made to **Figs 7A** to **7D** showing DSPSFL embodiments **300A - 300C.** Within the interior lumen of catheter member **310,** mandrel **312** is disposed, its ends shown extending throughout the entire length of catheter member **310,** in order to confer a required degree of firmness to DSPSFL **300A;** thereby providing for conveniently manipulating expandable flange **314A** at the distal end of catheter member **310** through a perforation in the cardiovascular system and in the heart. The interior lumen of catheter portion **310** is designed to allow sufficient amounts of liquids to pass through in a relatively short period of time and characterised by flow rates of at least 0.25 - 0.5 L/min. Mandrel **312** may be furnished with a blood indicator, as denoted above. Expandable flange **314A** retained in the contracted conformation by means of a cannula member (not shown) comprising a confining sleeve (not shown) threaded thereon and preventing expandable flange **314A** from unfolding into the expanded or sprawled conformation **314B.** The cannula member (not shown) and the confining sleeve (not shown) are characterized as cannula member **30** and sleeve **31** shown in **Figs 1** to **4****.** Expandable flange **314A** may be folded in the confining sleeve (not shown) in an umbrella-like manner. It should be acknowledged that the orientation of expandable flange **314A** folded in the proximal direction is merely exemplary; whereas a folding of expandable flange **314A** in the distal direction is equally applicable and may be preferred.

In some examples expandable flange **314A** is furnished with a scaffolding, such as a biasing string (not shown) threaded into a circumferential channel (not shown) embedded in the expandable flange **314A;** thus conferring the desired intrinsic bias and expandability to flange **314A.** Expandable flange **314A** may further include at least one structural element (not shown), in a non-limiting manner including slot, aperture, tab or pocket, dedicated for engagement with the tip of mandrel **312;** whereby upon of mandrel **312** expandable flange **314A** is respectively manipulated/advanced, similarly to what has been described hereinabove.

Proximally to expandable flange **314A,** on catheter member **310,** mitral washer **316,** inflatable toroidal cushion **318** and bushing **320** are disposed, being contiguously slidable therealong, collectively forming the sealing element of the DSPSFL. The inflatable lumen of toroidal member **318** forms a continuum with conduit **324** which is connected to inflator unit **328.** Examples of inflator unit **328** include: check valves, baffles, Luer tip actuated valves, Luer tip connectors, manually powered pumps, non-manually powered pumps, a pressurized gas tank and various combinations thereof. Above mitral washer **316,** inflatable toroidal cushion **318** and bushing **320** on catheter portion **310** clamp **330** is disposed, providing for contiguously driving the aforementioned elements along catheter portion **310** towards the expandable flange **314A** and securing them at the distal end thereof. Clamp **330** also provides for controlling the flow maintained through the interior lumen of catheter portion **310.**

Inflatable toroidal cushion **318** comprises a balloon-like lumen facing the biological tissue surrounding the perforation. This balloon-like lumen can be inflated, once the sealing element has been put in place, in order to create increased and more uniform pressure on the biological tissue surrounding the perforation thus facilitating more an efficient anastomosis.

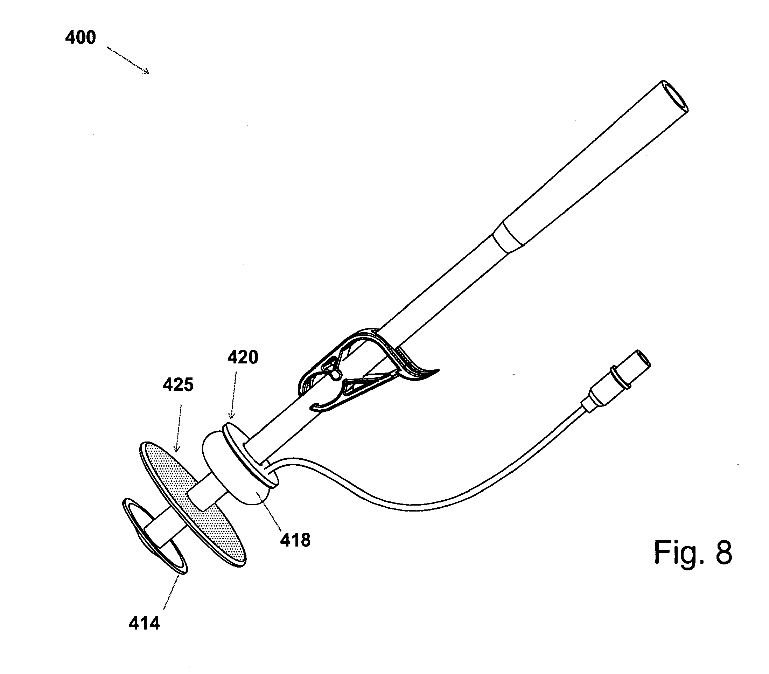

Reference is now made to **Fig. 8** wherein another preferred embodiment **400** of the DSPSFL of the present invention is shown. Embodiment **400** is in essence similar to the embodiment shown throughout **Figs. 7A - 7C****,** having similar expandable flange **414** and sealing element formed by inflatable toroidal cushion **418** and bushing **420.** An absorbent patch **425** is disposed in-between expandable flange **414** and expandable flange. Absorbent patch **425** can be covered, impregnated, embedded with or somehow otherwise contain either of the following agents: a super absorbent polymer (SAP); a coagulating and/or vasoconstrictive agent/s; an antiseptic, antibacterial or antifungal agent/s; anti-adhesive agents or coverings; anaesthetic or analgesic agents/s. SAP or some other applicable swelling materials known in the art may be contained within the absorbent patch to swell and increase the bulkiness thereof upon absorbing of fluids, such as blood, thereby facilitating an enhanced counterpressure against the area of biological tissue surrounding the perforation. Coagulating and/or vasoconstrictive agents, the latter include the examples of angiotensin and vasopressin, or any other of numerous suitable substances known in the art, may be contained within the absorbent patch to biochemically modify the blood or the surrounding biological tissue, thus intrinsically reducing the haemorrhage. Antiseptic, antibacterial or antifungal agents include numerous examples of suitable substances known in the art that are capable to prevent or reduce a bacterial or fungal infection. Anaesthetic or analgesic agents can be any of the numerous suitable substances known in the art that provide a relief from the pain and discomfort that may be experienced by the injured. Anti-adhesive agents or coverings preventing patch's adhering to the biological tissue surrounding the perforation include the example of TELFA™ Island Dressings, item code 7665, available from Tyco Healthcare / Kendall, 15 Hampshire Street, Mansfield, Massachusetts US.

According to the best mode of carrying out the invention, in some preferred embodiments, the expandable flange and/or sealing element and/or at least the distal portion of the catheter member are made of biodegradable materials, thereby providing for suturing these components into the living tissues of the patient and remaining to degrade therein.

The expandable flange, as well as the catheter member, can assume a variety of forms, shapes and sizes and be made of various materials, all in order to suit the specific implementation of the DSPSFL and comply with the requirements of the medical condition to be treated. Thus, for instance, the size can vary in order to cover a range of perforations of different sizes and/or to comply with the anatomical constraints associated with different body parts to be treated or in order to accommodate different types of injuries, perforations, raptures or incisions.

In view of the foregoing, it is stressed that all the variations of the expandable flange and/or the catheter member, regardless of their particular form, shape, size, materials they are made of are considered to be within the scope of the present invention, provided that the expandable flange is located at the distal end of the catheter member and inserted through a perforation rapture or incision into a organ to be treated/sealed, wherein the expandable flange assumes its expanded sprawled or unfolded conformation.

The sealing element can take a variety of forms, shapes and sizes and can be made of various materials, all in order to suit the specific implementation of the device and comply with the requirements of the medical condition to be treated. Thus, for instance the size of the sealing element can vary in order to cover a range of perforations of different sizes and or to suit the anatomic sites to be treated with the device and the shape can vary in order to suit different kind of injuries.

In view of the foregoing, it should be stressed that all the variations of the sealing element, regardless of their particular form, shape, size or materials they are made of, are considered to be within the scope of the present invention, provided that the condition that the sealing element is pressed against the expandable flange, onto the biological tissue surrounding the perforation, from the outside of the organ treated/sealed.

### Operation of the device of the invention

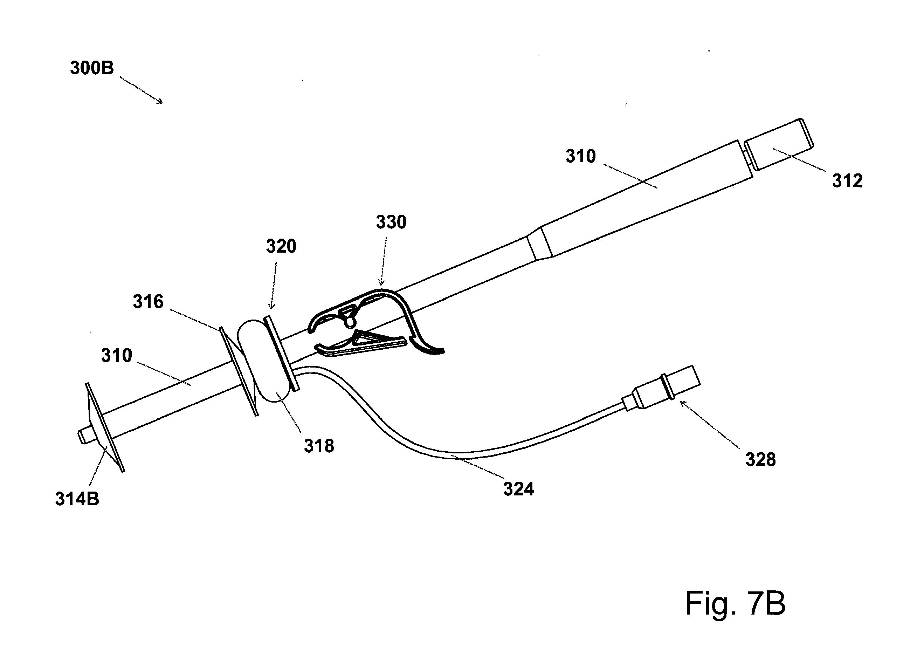

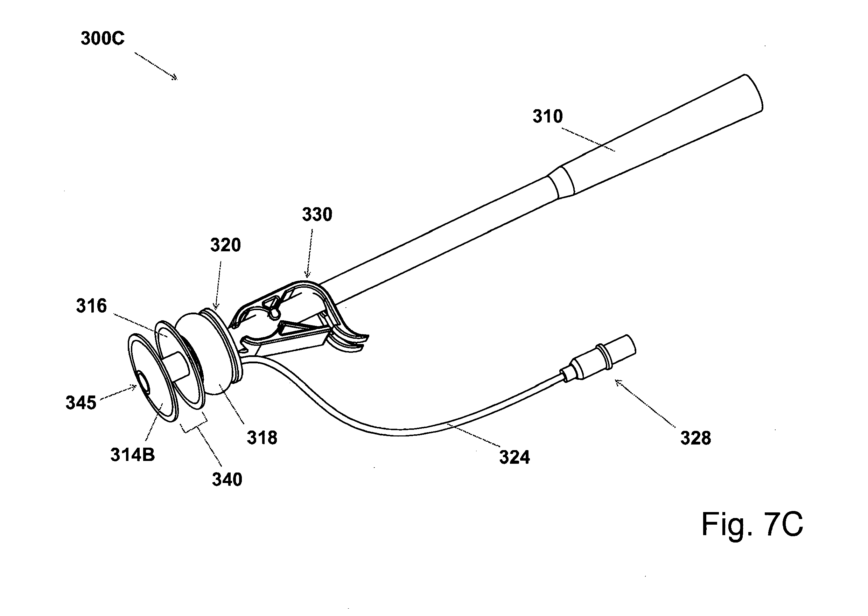

It is noted that the method of operating the embodiment of DSPSFL adapted for sealing perforations in an essentially tubular organs, shown in **Figs 1** to **4** and **Figs 5** to **6****,** is explained below by the way of example of operating the DSPSFL dedicated for globular or apical organs, shown in **Figs 7** to **8****.** Accordingly the references and numerals of particular parts or portions of the DSPSFL and/or the subject organ/s it is implemented on should be construed mutatis mutandis and interchangeably with their respective equivalents and counterparts of DSPSFL shown in **Figs 1** to **6****.**

DSPSFL **300A** is typically packed in a sterile packaging, while the cannula member (not shown) is anteriorly disposed on catheter member **310;** thereby confining expandable flange **314A** and preventing the sprawling/unfolding thereof, i.e. retaining it in the contracted or folded conformation. In the contracted or folded conformation the effective exterior diameter of DSPSFL **300A** is the outer diameter of the cannula member (not shown) and the anterior end is essentially the tip of the confining sleeve (not shown), occluded by mandrel **312** inserted thereto and expandable flange **314A** folded therein. The sealing element is disposed on the confining sleeve (not shown) towards the posterior portion of DSPSFL **300A.**

In the instance of DSPSFL adapted for sealing perforations in essentially tubular organs, shown in **Figs 1** to **4** and **Figs 5** to **6****,** the concave interior surface of expandable flange **16** is preferably disposed inside the cannula vis-à-vis the pointed end of tip **32.** The aforementioned orientation of the concave interior surface of expandable flange **16** is disposed inside the cannula vis-à-vis the pointed end of tip **32,** provides for inserting the pointed end of tip **32** into the perforation first and then deploying expandable flange **16** so that the convex exterior face thereof facing the perforation.

The operator appends DSPSFL **300A** to the perforation, rapture or incision to be sealed. If the perforation is formed within a deep wound, the operator may introduce the anterior portion of DSPSFL **300A,** namely anterior portion of confining sleeve (not shown) thereto, until the tip of the cannula member (not shown) is put adjacently to the perforation, rapture or incision. The operator then, by manipulating posterior end of DSPSFL **300A** and particularly the proximal cap of mandrel **312,** inserts the tip of the cannula member (not shown) throughout the perforation or incision into the organ to be sealed. Provided that the organ to be sealed is a part of the cardiovascular system, the operator may refer to the blood indicator, to verify the presence of the tip of mandrel **312** in a positive blood pressure environment.

Following the insertion of the tip of the cannula member (not shown) operator deploys expandable flange **314A** inside the organ to be sealed, by purposefully pulling the tab of the cannula member over catheter member **310** while stabilizing the posterior cap of mandrel **312.** Upon pulling the tab of the cannula member (not shown), the confining sleeve (not shown) is posteriorly retracted and the tip of the cannula member is slid away over expandable flange **314A;** thereby flange **314A** and the anterior portion of the catheter member **310** are exposed. Consequently expandable flange **314A** spontaneously unfolds/sprawls, thus assuming an expanded sprawled or unfolded conformation, such as flange **314B,** inside the organ. To completed the deployment of expandable flange **314B** within the organ, the operator may slightly pull DSPSFL **300A** in a posterior direction, so that the proximal face of the flange **314B,** e.g. the convex face of expandable flange **16,** adjoins an area of biological tissue surrounding the perforation from inside the organ, e.g. the concave face of interior of a blood vessel.

After expandable flange **314B** was deployed in the organ to be sealed by assuming an expanded sprawled or unfolded conformation therein, mandrel **312** can be removed by being posteriorly retracted and eventually pulled out of the interior lumen of catheter member **310.** Subsequently clamp **330** can be compressed to prevent an outflow from the outlet of DSPSFL **300B.** By applying a manual force to clamp **330,** slidably driving it along catheter member **310,** the sealing element is urged towards expandable flange **314B,** so that an area of biological tissue surrounding the perforation is captured within gap **340,** namely in-between flange **314B** and the sealing element, as schematically shown in **Fig. 7C****.** Inflatable toroidal cushion **318** is thence can be inflated facilitating an enhanced sealing of the perforation; simultaneously the inflation of toroidal member **318** affixes it to the exterior surface of catheter member **310.**

Upon exposure to a positive pressure environment inside the organ, the thin-walled neck portion of the catheter member, such as neck portion **14,** expands, thus adjoining the edges of the perforation or incision, thereby facilitating an enhanced sealing thereof; whereas the infinitesimal intrinsic bias of the thin-walled neck portion of the catheter member does not forcefully spreads the tissue of the perforation, thus providing for the closure thereof.

The inlet of DSPSFL **300C** at proximal end of catheter member **310** can then be connected to a source of infusion medium, which can be perfused via the interior lumen of catheter member **310** into the organ through outlet **345** at the distal end thereof. The lumen of catheter **member 310** allows sufficient amounts of liquids to pass through in a relatively short period of time and characterised by flow rates of at least 0.25 - 0.5 Umin.

### Scope of use of the of the invention

The device of the present invention is inter alia intended for an initial emergency field-care, typically performed within the vicinity of the site and with proximity to the time of the injury incident occurrence in order to substantially reduce a haemorrhage, avoid exsanguination, and stabilize injurer's condition for further transportation to a medical facility. Nevertheless, the device's usage is not limited to emergency field-care of injury incidents, thus the device of the present invention is equally applicable for all perforations and raptures of the blood vessels and/or heart walls regardless the nature of the phenomena that caused such perforation or rupture, which is of a noticeable clinical benefit to the patient. Instances of such other perforations and ruptures include: a spontaneous rupture that may occur in the walls of a heart or abdominal aorta or iatrogenic trauma caused in a surgery room.

Additional application of the DSPSFL of the present invention is the performing of a quick aortic cannulation procedure, by obviating the purse-string sutures for affixing the cannula. Such rapid cannulation can be beneficially employed for achieving a cardiopulmonary bypass to heart-lung life support systems, as promptly as possible. If the DSPSFL is employed for aortic cannulation, the DSPSFL is typically deployed through an incision. The embodiments of the device to be used as aortic cannulae shall have accordingly designed expandable flange and sealing element. Shape, size and other physical properties of the expandable flange and sealing element may also vary to meet the requirements of implementing the device in a particular cannulation procedure or particular site of cannulation, such as ascending or descending aorta.

It should be acknowledged, however, that the implementation of DSPSFL is not limited to the cardiovascular system, e.g. blood vessels or the heart. The DSPSFL can be beneficially implemented of various organs and/or tracts and employed in various medical applications. Thus the DSPSFL can be beneficially implemented on any anatomic passage, cavity or tubuloalveolar structure formed within a body or an organ, such as the gastrointestinal tract, respiratory tract, urinary tract and male or female reproductive tract, including the esophagus, stomach, small intestine and duodenum colon, trachea, pleura/pleural cavity, lungs, urethra, ureters and bladder. The DSPSFL can also be employed as a trocar for draining liquids or gases from the sealed organ. An application of the DSPSFL as a trocar can be exemplified by intercostal tube thoracostomy treatment of pneumothorax.

The necessity in the device of the present invention can be emphasized by the long lasting unsatisfied need therefor; since hitherto physicians and paramedics occasionally used to improvise with Foley catheters in lieu of a dedicated device, in order to seal perforations occurred in the ventricles or atriums of a heart. However, Foley catheters are less then suboptimal for the task achieved by device of the present invention, since the size and the spherical shape of the inflatable balloon of the Foley catheter would completely block any blood vessel and fail to achieve an efficient anastomosis.

It will be appreciated that the present invention is not limited by what has been particularly described and shown hereinabove and that numerous modifications, all of which fall within the scope of the present invention, exist. Rather the scope of the invention is defined by the claims which follow:

## Claims

1. A medical device for at least sealing perforations comprising:
[a] a catheter member (12) comprising at least an expandable flange (16) at the distal end thereof, wherein said flange (16) is capable of assuming at least one conformation selected from the group consisting of: a contracted conformation, folded conformation, expanded conformation, sprawled conformation and unfolded conformation;
[b] a mandrel member (20), wherein said mandrel member (20) is insertable into said catheter member (12);
[c] a cannula member (30), wherein said cannula member (30) is disposable over said catheter member (12);
[d] a sealing element (40);
wherein said sealing element is threadable onto at least one selected from the group consisting of: said catheter member (12) and said cannula member (30).

2. A medical device as in claim 1, wherein said expandable flange (16) is retained in said contracted conformation or said folded conformation by said cannula member (30).

3. A medical device as in claim 1, wherein said catheter member (12) further comprises a relatively thin-walled neck portion adjacently to said flange (16).

4. A medical device as in claim 1, wherein said sealing element comprises at least one selected from the group consisting of: a bushing (42), toroidal cushion (44), inflatable lumen and conduit.

5. A medical device as in claim 1, wherein said expandable flange (16) is **characterized by** a trough-shaped configuration, wherein the convex surface thereof is adapted to adjoin the interior concave surface of an essentially tubular organ.

6. A medical device as in claim 1, wherein said expandable flange (16) comprises an outlet aperture (17) forming a continuum with an interior lumen of said catheter member (12).

7. A medical device as in claim 1, wherein said expandable flange (16) comprises at least one structural element dedicated for engagement with said mandrel member (20).

8. A medical device as in claim 1, wherein said mandrel member (20) comprises at least one structural element dedicated for engagement with said expandable flange (16).

9. A medical device as in claim 1, wherein said mandrel (20) comprises a blood indicator.

10. A medical device as in claim 1, wherein a tip (32) of said cannula member (30) is shaped and/or sized as a tip of an aortic cannula.

## Patentansprüche

1. Medizinische Vorrichtung zumindest zum Abdichten von Perforationen, aufweisend:
[a] ein Katheterelement (12), das zumindest einen erweiterbaren Flansch (16) an seinem distalen Ende aufweist, wobei der Flansch (16) in der Lage ist, mindestens eine Konformation anzunehmen, die ausgewählt ist aus der Gruppe bestehend aus: einer kontrahierten Konformation, einer gefalteten Konformation, einer erweiterten Konformation, einer gespreizten Konformation und einer entfalteten Konformation;
[b] ein Dornelement (20), wobei das Dornelement (20) in das Katheterelement (12) eingeführt werden kann;
[c] ein Kanülenelement (30), wobei das Kanülenelement (30) über dem Katheterelement (12) angeordnet werden kann;
[d] ein Abdichtungselement (40);
wobei das Abdichtungselement auf mindestens ein Element der aufgeschraubt werden kann, das ausgewählt ist aus der Gruppe, die besteht aus: dem Katheterelement (12) und dem Kanülenelement (30).

2. Medizinische Vorrichtung nach Anspruch 1, wobei der erweiterbare Flansch (16) durch das Kanülenelement (30) in der kontrahierten Konformation oder der gefalteten Konformation gehalten wird.

3. Medizinische Vorrichtung nach Anspruch 1, wobei das Katheterelement (12) ferner einen relativ dünnwandigen Halsabschnitt angrenzend an den Flansch (16) aufweist.

4. Medizinische Vorrichtung nach Anspruch 1, wobei das Abdichtungselement mindestens eines umfasst, das ausgewählt ist aus der Gruppe bestehend aus: einer Hülse (42), einem ringförmigen Dämpfer (44), einem aufblasbaren Lumen und einer Leitung.

5. Medizinische Vorrichtung nach Anspruch 1, wobei der erweiterbare Flansch (16) durch eine muldenförmige Gestaltung gekennzeichnet ist, wobei deren konvexe Oberfläche für die Anlage an die konkave Innenfläche eines im Wesentlichen röhrenförmigen Organs ausgelegt ist.

6. Medizinische Vorrichtung nach Anspruch 1, wobei der erweiterbare Flansch (16) eine Auslassöffnung (17) aufweist, die ein Kontinuum mit einem Innenlumen des Katheterelements (12) bildet.

7. Medizinische Vorrichtung nach Anspruch 1, wobei der erweiterbare Flansch (16) mindestens ein strukturelles Element umfasst, das für einen Eingriff mit dem Dornelement (20) vorgesehen ist.

8. Medizinische Vorrichtung nach Anspruch 1, wobei das Dornelement (20) mindestens ein strukturelles Element aufweist, das für einen Eingriff mit dem erweiterbaren Flansch (16) vorgesehen ist.

9. Medizinische Vorrichtung nach Anspruch 1, wobei das Dornelement (20) einen Blutindikator umfasst.

10. Medizinische Vorrichtung nach Anspruch 1, wobei eine Spitze (32) des Kanülenelements (30) der Form und/oder der Größe nach wie die Spitze einer Aortakanüle ausgebildet ist.

## Revendications

1. Un dispositif médical destiné à au moins sceller des perforations comprenant :
[a] un élément cathéter (12) comprenant au moins une bride extensible (16) au niveau de l'extrémité distale de celui-ci, où ladite bride (16) est capable d'adopter au moins une conformation sélectionnée dans le groupe se composant de : une conformation contractée, une conformation pliée, une conformation étendue, une conformation étalée et une conformation non pliée,
[b] un élément mandrin (20), où ledit élément mandrin (20) peut être inséré dans ledit élément cathéter (12),
[c] un élément canule (30), où ledit élément canule (30) peut être disposé sur ledit élément cathéter (12),
[d] un élément de scellement (40),
où ledit élément de scellement peut être vissé dans au moins un élément sélectionné dans le groupe se composant de : ledit élément cathéter (12) et ledit élément canule (30).

2. Un dispositif médical selon la Revendication 1, où ladite bride extensible (16) est maintenue dans ladite conformation contractée ou ladite conformation pliée par ledit élément canule (30).

3. Un dispositif médical selon la Revendication 1, où ledit élément cathéter (12) comprend en outre une partie encolure à paroi relativement mince adjacente à ladite bride (16).

4. Un dispositif médical selon la Revendication 1, où ledit élément de scellement comprend au moins un élément sélectionné dans le groupe se composant de : une bague de raccordement (42), un coussin toroïdal (44), un conduit et un lumen gonflables.

5. Un dispositif médical selon la Revendication 1, où ladite bride extensible (16) est **caractérisée par** une configuration en forme d'auge, où la surface convexe de celle-ci est adaptée de façon à être attenante à la surface concave intérieure d'un organe essentiellement tubulaire.

6. Un dispositif médical selon la Revendication 1, où ladite bride extensible (16) comprend une ouverture de sortie (17) formant un continuum avec un lumen intérieur dudit élément cathéter (12).

7. Un dispositif médical selon la Revendication 1, où ladite bride extensible (16) comprend au moins un élément de structure dédié à une mise en prise avec ledit élément mandrin (20).

8. Un dispositif médical selon la Revendication 1, où ledit élément mandrin (20) comprend au moins un élément de structure dédié à une mise en prise avec ladite bride extensible (16).

9. Un dispositif médical selon la Revendication 1, où ledit mandrin (20) comprend un indicateur sanguin.

10. Un dispositif médical selon la Revendication 1, où une pointe (32) dudit élément canule (30) est façonnée et/ou dimensionnée sous la forme d'une pointe d'une canule aortique.
